# FASCICULE DE BREVET EUROPEEN

(11) **EP 0 663 903 B1**
(45) Date de publication et mention de la délivrance du brevet: **23.04.1997**
(21) Numéro de dépôt: 93922005.9
(22) Date de dépôt: 07.10.1993
(51) Int. Cl.: C07D 241/44, A61K 31/495

(54) **DERIVES DE DIHYDRO-1,2 OXO-2 AMINO-3 QUINOXALINES, LEUR PREPARATION ET LEUR APPLICATION EN THERAPEUTIQUE**
1,2-DIHYDRO-2-OXO-3-AMINOCHINOXALIN DERIVATE, IHRE VERWENDUNG ALS HEILMITTEL
1,2-DIHYDRO-2-OXO-3-AMINO QUINOXALINE DERIVATIVES, PREPARATION THEREOF AND APPLICATION IN THERAPY

(30) Priorité: 07.10.1992 FR 9211878
(43) Date de publication de la demande: 26.07.1995
(73) Titulaire: PIERRE FABRE MEDICAMENT, 92100 Boulogne (FR)
(72) Inventeur: BIGG, Dennis, F-81100 Castres (FR); PATOISEAU, Jean-François, F-81100 Castres (FR); AUTIN, Jean-Marie, 81290 Labruguière (FR); TARAYRE, Jean-Pierre, F-81100 Valdurenque (FR)
(74) Mandataire: Ahner, Francis
(86) Numéro de dépôt international: FR9300991
(87) Numéro de publication internationale: WO9407870

(56) Documents cités:
- EP-A- 0 008 864
- JOURNAL OF THE CHEMICAL SOCIETY no. 78 , 1957 , LETCHWORTH GB pages 422 - 430 J. CLARCK-LEWIS 'QUINOXALINE DERIVATIVES.PART III.'

## Description

La présente invention , réalisée au Centre de Recherche PIERRE FABRE MEDICAMENT, a pour objet de nouveaux composés chimiques, leur procédé de préparation et leur application en tant que médicaments.

Les synthèses de dihydro-1,2 oxo-2 quinoxalines diversement fonctionnalisées en position 3 sont reportées dans la littérature. Parmi ces composés on peut noter des esters (F.E. KING et J.W. CLARK-LEWIS, J. Chem. Soc. 1953, 172-177), des amides (H. BREDERECK et W. PFLEIDERER, Chem. Ber. 87, 1119-23 (1954) ou des amines. Dans cette catégorie, E. SCHIPPER et A.R. DAY décrivent la synthèse de la dihydro-1,2 amino-3 oxo-2 quinoxaline (J. Am. Chem. Soc. 73, 5672-5675 (1951)) et J.W. CLARK-LEWIS celle de la dihydro-1,2 aminométhyl-3 méthyl-1 oxo-2 quinoxaline (J.Chem. Soc. 1957, 422). Par ailleurs, la présence en position 3, d'une chaîne carbonée, fonctionnalisée ou non, confère aux molécules une activité bronchodilatatrice, revendiquée dans les brevets suivants :
- US, 4.181.724 du 1.1.80, Appl. 940.815 du 11.9.78 (UPJOHN. Co).
- Demande FR 89.13961 du 23.10.89 (Pierre FABRE Médicament).

La présente invention concerne les composés de formule générale I 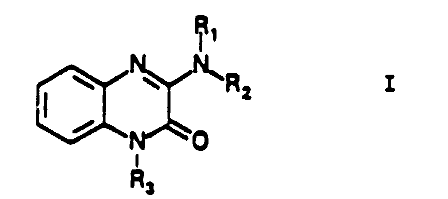 dans laquelle
- R₁ et R₂: représentent, indépendamment l'un et l'autre, l'hydrogène ou un radical alcoyle en C₁-C₄ linéaire ou ramifié,
- R₃: représente un groupement alcoyle, alcényle ou hydroxyalcoyle, en C₃-C₆ linéaire ou ramifié.

En outre, l'invention couvre les sels des composés de formule générale I avec des acides pharmaceutiquement acceptables dans le cas de composés présentant une basicité suffisante.

Les composés de formule générale I de l'invention peuvent être préparés selon le schéma de réaction suivant :

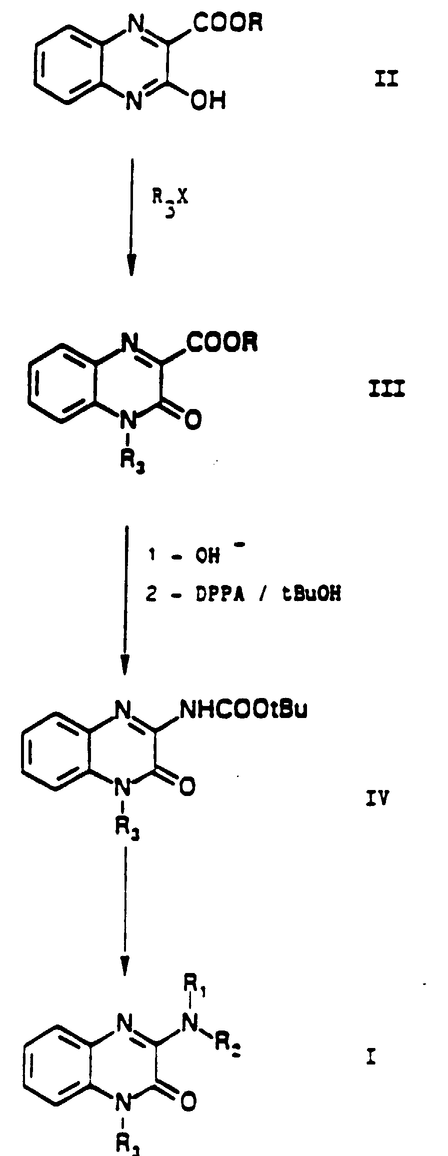

Les composés de départ de formule générale II dans lesquels R représente méthyle ou éthyle peuvent être obtenus selon des méthodes connues, par exemple celles décrites par ABDULLA et al. J. Heterocyclic. Chem., 13,427 (1976) ou par SEN et al., J. Indian Chem. Soc., Vol 38, n° 4,1961, p. 225-228. L'hydroxyester II est traité par un réactif R₃S dans lequel R₃ a la même signification que précédemment et X représente un groupement nucléofuge tel que l'atome d'iode, de chlore ou de brome ou un groupe mésylate ou tosylate. La réaction peut être effectuée en présence d'une base telle que l'hydrure de sodium au sein d'un solvant aprotique polaire tel que le DMF. Elle peut également être réalisée dans un milieu tel que K₂CO₃-acétone. Après saponification à la soude ou à la potasse en milieu alcoolique, le composé III obtenu, pour lequel R représente un atome d'hydrogène, est traité par le diphénylphosphoryle azide dans le tertiobutanol en présence d'une base telle que la triéthylamine. La réaction est conduite de préférence à une température comprise entre 50°C et la température d'ébullition du tertiobutanol pour former, via l'isocyanate intermédiaire non isolé, le composé IV.

Le composé IV est traité
- soit par un composé R₁Y, R₁ représentant un groupe alcoyle en C₁-C₄ linéaire ou ramifié et Y représentant un groupement labile tel que halogène (Cl, Br, I), mésylate ou tosylate.
- soit par un dialcoyle C₁-C₂ sulfate,
puis soumis à une hydrolyse acide pour conduire au composé I (R₁ ≠ H, R₂ = H).

La réaction d'alcoylation est avantageusement effectuée dans des conditions de transfert de phase en utilisant un solvant non miscible à l'eau tel que le THF et un catalyseur de transfert de phase tel que, à titre d'exemple le chlorure de benzyl triéthyl ammonium. L'hydrolyse acide peut être réalisée par traitement à l'acide chlorhydrique alcoolique à une température comprise entre la température ambiante et la température d'ébullition du solvant, ou à l'acide trifluoroacétique à température ambiante. Le même composé IV est directement soumis à hydrolyse acide pour accéder au composé I dans lequel R₁ = R₂ = H.

Les composés de formule générale I pour lesquels R₁ et/ou R₂ représentent l'hydrogène peuvent être alcoylés selon les méthodes classiques utilisées pour alcoyler les amines primaires ou secondaires tel que, à titre d'exemple, le traitement par un halogénure d'alcoyle en C₁-C₄, pour conduire aux composés I dans lesquels R₁ et R₂ ≠ H.

Les exemples ci-après illustrent l'invention sans toutefois en limiter la portée.

Les analyses centésimales ainsi que les spectres infrarouge et RMN confirment la structure des composés obtenus.

### Exemple 1

1) n-propyl-1 dihydro-1,2 oxo-2 quinoxaline carboxylate d'éthyle-3 1.
   A une suspension de 18,25 g (0,084 mole) d'hydroxy-2 quinoxaline carboxylate d'éthyle-3 dans 300 ml d'acétone, on ajoute 11,55 g (0,084 mole) de carbonate de potassium, 22,8 ml de bromure de n-propyle et on chauffe le tout 7 heures à reflux. Après évaporation du solvant sous vide, la masse résiduelle est extraite à l'éther éthylique. Les sels minéraux sont alors filtrés et le filtrat évaporé sous vide. L'huile brune ainsi obtenue est purifiée sur colonne de silice. L'élution par un mélange acétate d'éthyle-hexane 30-70 fournit, après évaporation, 14,6 g (rendement 58 %) de composé 1 sous forme de cristaux jaunes (F = 65°C). CCM : Rf = 0,35 (acétate d'éthyle - hexane 30-70).
2) Acide n-propyl-1 dihydro-1,2 oxo-2 3 quinoxaline carboxylique-3 2.
   Une solution de 14,6 g d'ester 1 (0,056 mole) dans 50 ml d'éthanol à 95° et 56 ml de soude 2N (0,112 mole) est agitée 30 minutes à 50°C. Après refroidissement et acidification par addition d'une solution d'acide chlorhydrique N, la suspension obtenue est glacée pendant une heure. Les cristaux sont alors filtrés, lavés à l'eau, à l'éthanol à 95° puis séchés sous vide à 50°C. On récupère ainsi 12,24 g d'acide 2 sous forme de paillettes jaunes (rendement 94 %). F = 172°C
   CCM = Rf = 0,65 (méthanol - chloroforme 50-50).
3) n-propyl-1 dihydro-1,2 oxo-2 terbutoxycarbonyl amino-3 quinoxaline 3.
   Une solution de 12,24 g (0,05227 mole) de l'acide 2, 15,1 ml (0,11 mole) de triéthylamine, 23,1 ml (0,105 mole) de diphényl phosphorylazide dans 350 ml de terbutanol est chauffée pendant 5 heures à reflux. Après évaporation du solvant, l'huile résiduelle est reprise par une solution de bicarbonate de sodium et extraite à l'acétate d'éthyle.
   La phase organique est lavée à l'eau puis à l'eau salée, puis séchée sur sulfate de sodium et concentrée sous vide. Le solide obtenu est trituré dans l'éther éthylique, filtré, lavé et séché pour donner 12 g d'aiguilles blanches de composé 3 (rendement 75 %).
   F = 126-127°C.
   CCM = Rf : 0,61 (acétate d'éthyle - hexane 30-70).
4) n-propyl-1 dihydro-1,2 oxo-2 amino-3 quinoxaline 4.
   Une solution de 7,12 (0,0234 mole) de carbamate 3 dans 50 ml de méthanol et 35 ml d'acide chlorhydrique 6N est agitée 4 heures à température ambiante. On neutralise, sous refroidissement, par addition de soude 6N. Les cristaux formés sont filtrés, lavés à l'eau et séchés à 50°C. On obtient ainsi 4,55 g (rendement 95 %) de composé 4 sous forme d'une poudre blanche.
   F = 191-192°C.
   CCM = Rf : 0,65 (acétate d'éthyle - hexane 50-50).
5) n-propyl-1 dihydro-1,2 oxo-2 amino-3 quinoxaline; chlorhydrate 5.
   Les cristaux de composé 4 obtenus ci-dessus sont mis en suspension dans l'éthanol. On ajoute ensuite une solution éthanolique de gaz chlorhydrique : la base se dissout puis le chlorhydrate commence à précipiter. Après refroidissement et dilution à l'éther éthylique, les cristaux sont filtrés puis lavés avec un mélange éthanol-éther éthylique 50-50 puis à l'éther éthylique. Après séchage, on récupère 4,25 g (rendement 80 %) de composé 5 sous forme de cristaux blancs.
   F = 188°C (sublimation).
   CCM = Rf : 0,65 (acétate d'éthyle - hexane 50-50).

### Exemple 2

n-propyl-1 dihydro-1,2 oxo-2 méthylamino-3 quinoxaline; chlorhydrate 6.

Une solution de 9,32 g (0,03 mole) de carbonate 3 dans 100 ml de tétrahydrofurane est agitée 24 heures à température ambiante en présence de 3,4 ml de sulfate de méthyle, 21 ml de soude 6N et 684 mg de chlorure de benzyl triéthyl ammonium.

Après évaporation du solvant, le résidu est repris à l'eau et extrait à l'acétate d'éthyle. Après lavage à l'eau, séchage sur sulfate de sodium et évaporation sous vide, l'huile récupérée (N-méthyl carbamate intermédiaire) est traitée par une solution de 50 ml d'acide chlorhydrique 6N pendant 2 heures à 60°C. Cette solution est ensuite versée sur de la glace et neutralisée par addition de soude 6N. Le solide formé est extrait à l'acétate d'éthyle et le chlorhydrate isolé en opérant de façon identique à l'exemple précédent.

On récupère ainsi 3,30 g (rendement 65 %) de composé 6 sous forme de cristaux blancs;
F = 150-151°C.
CCM = Rf : 0,50 (acétate d'éthyle - hexane 30-70).

### Exemple 3

n-propyl-1 dihydro-1,2 oxo-2 diméthylamino-3 quinoxaline ; chlorhydrate 7.

On agite pendant 1 heure à température ambiante 2 g (0,009 mole) de composé 6 dans 15 ml de N,N diméthylacétamide en présence de 0,4 g d'hydrure de sodium à 60 %. On ajoute ensuite 1,54 ml (0,025 mole) d'iodure de méthyle et on chauffe 1 heure à 60°C.

Après évaporation du solvant sous vide, le chlorhydrate est obtenu de la même façon que dans les exemples précédents.

On isole 1,79 g de cristaux blancs de composé 7 (rendement = 75 %).
F = 120-121°C.
CCM = Rf : 0,30 (acétate d'éthyle - hexane 10-90).

### Exemple 4

prenyl-1 dihydro-1,2 oxo-2 amino-3 quinoxaline ; chlorhydrate 14.

Une solution de 4 g de prenyl-1 dihydro-1,2 oxo-2 terbutoxy carbonyl amino-3 quinoxaline (0,012 mole) dans l'acide trifluoroacétique est laissée sous agitation pendant 30 minutes. Après évaporation sous vide, le résidu est repris par une solution de bicarbonate de sodium et extrait à l'acétate d'éthyle. La phase organique est ensuite lavée à l'eau, séchée sur sulfate de sodium et concentrée.

On obtient, après purification sur gel de silice, par élution avec un mélange acétate d'éthyle - CHCl₃ 30-70, puis après chlorhydratation de façon identique aux exemples précédents, 2,5 g de composé 14 sous forme de cristaux blancs (rendement 78 %).
F = 191°C.
CCM = Rf : 0,5 (acétate d'éthyle - hexane 50-50).

### Exemple 5

(méthyl-3 hydroxy-3) butyl-1 dihydro-1,2 oxo-2 amino-3 quinoxaline ; chlorhydrate 17.

Une solution de 4 g de prenyl-1-dihydro-1,2 oxo-2 terbutoxy carbonyl amino-3 quinoxaline (0,012 mole) dans 30 ml d'acide chlorhydrique 6N est portée pendant 2 heures à 80°C sous agitation.

Après refroidissement, la solution est versée sur de la glace et neutralisée par de la soude 6N. Le solide formé est, après filtration et lavage à l'eau, repris par l'acétone, puis la solution est séchée sur sulfate de sodium et évaporée à sec sous vide. On obtient, après chlorhydratation, 3 g de composé 17 (rendement 93 %) sous forme de cristaux blancs.
F = 190°C.
CCM = Rf : 0,25 (acétate d'éthyle - hexane 50-50).

Le tableau I ci-après résume les principaux produits synthétisés.

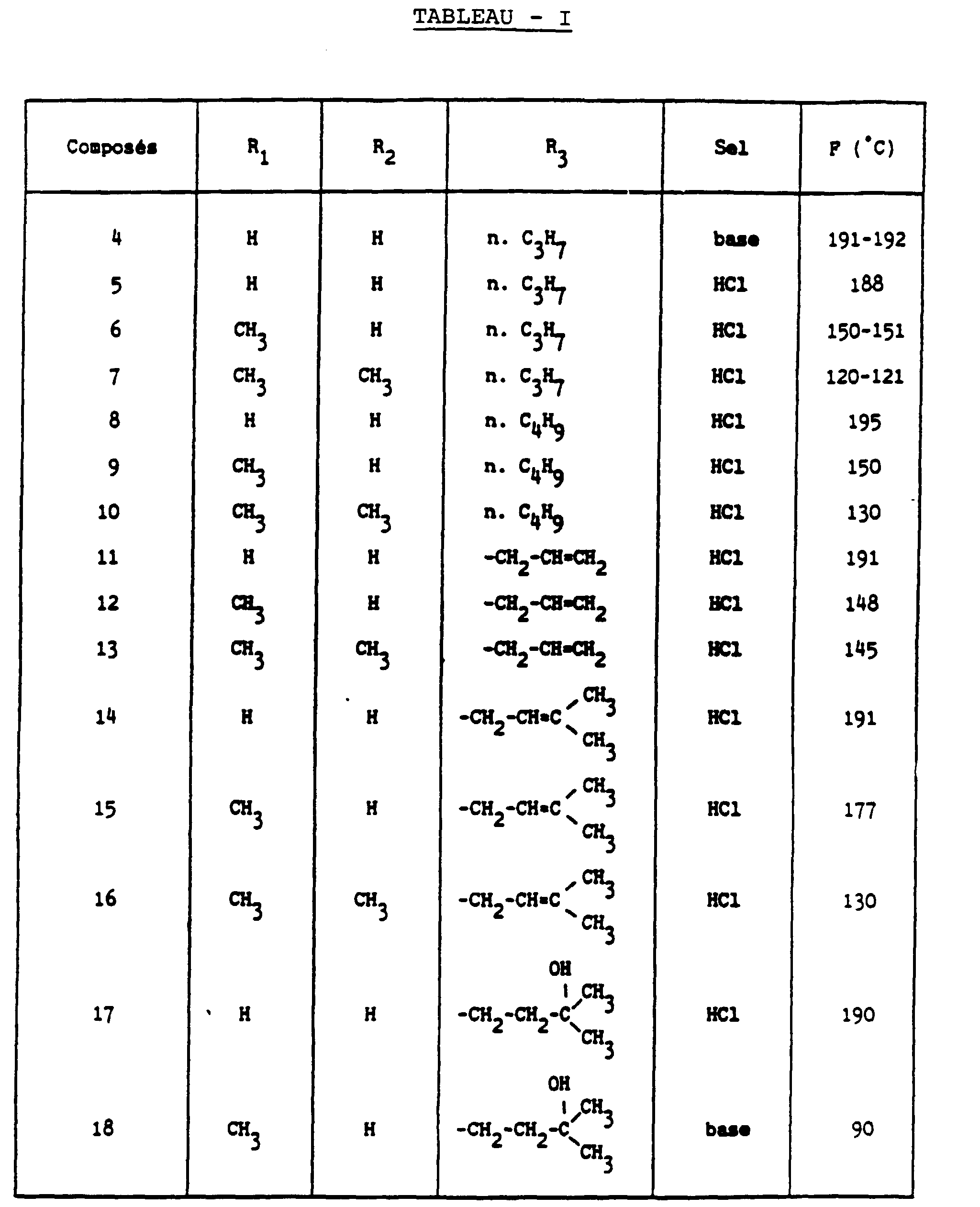

### EXPERIMENTATIONS BIOLOGIQUES

### 1 - Etude pharmacologique

Les composés de l'invention ont été soumis à des essais pharmacologiques qui ont montré leur intérêt en tant que broncholytiques.

A cet effet, les composés ont été étudiés pour leur effet antagoniste des contractions provoquées par divers produits selon le protocole suivant.

Un morceau de trachée de cobaye tricolore mâle de 400 g en moyenne est découpé en spirale et monté (2,5 cm environ) dans une cuve à organe isolé thermostatée à 37°C et remplie de tyrode oxygéné. On enregistre les contractions isométriques provoquées par les divers médiateurs grâce à un capteur UC2 Gould Statham (Gould Inc. Oxnard, California, USA) ou UF1 Palmer Bioscience relié à un enregistreur potentiométrique (Linseiss, Selb, RFA).

Au début de l'expérience, le lambeau de trachée est soumis à une tension de 1 g et laissé au repos durant 1 heure.

On utilise les concentrations suivantes de médiateurs (concentrations provoquant en général une contraction maximale) : dichlorhydrate d'histamine, 10 µg/ml; chlorure de potassium, 1850 µg/ml. Les concentrations d'agents utilisés entraînent des contractions qui deviennent maximales au bout de 5 à 15 mn suivant les médiateurs et se maintiennent en palier ensuite. Une concentration du produit étudié est administrée pour chaque contraction et le produit est laissé au contact de la trachée durant 5 mn. On mesure l'inhibition éventuelle obtenue au bout de ce temps (pourcentage de variation de l'amplitude de la contraction). La trachée est ensuite lavée durant 15 secondes et laissée au repos durant 10 mn environ (temps nécessaire au retour au tonus de base) avant de provoquer une nouvelle concentration.

Les produits insolubles dans l'eau sont dilués dans 0,178 % (v/v) (concentration finale dans le bain) de diméthyl sulfoxide (DMSO). Cette concentration de DMSO n'entraîne aucun effet vis-à-vis des contractions des divers médiateurs étudiés.

Les concentrations inhibitrices 50 % (IC₅₀) sont calculées en utilisant un programme SAS (Statistical Analysis System) inspiré de Bliss et Cattel (BLISS C.I. et CATTEL McK. Biological Assay Ann. Rev. Physiol. 5, 479, 1943).

Les résultats obtenus sur certains composés de l'invention sont reportés, à titre d'exemple, dans les tableaux 2 et 3.

**TABLEAU - II**

| ANTAGONISME DE L'EFFET DU CHLORURE DE POTASSIUM | |
|---|---|
| Composé n° | IC₅₀ (µg/ml) |
| 5 | 19,3 |
| 6 | 4,7 |
| 7 | 28 |
| 8 | 9,6 |
| 9 | 11,1 |
| 10 | 45 |
| 11 | 21 |
| 12 | 9,1 |
| 13 | 19,3 |
| 14 | 7,4 |
| 17 | 45 |
| Théophylline | 30 |

**TABLEAU - III**

| ANTAGONISME DE L'EFFET DE L'HISTAMINE | |
|---|---|
| Composé n° | IC₅₀ (µg/ml) |
| 5 | 4,6 |
| 6 | 6,4 |
| 7 | 10,7 |
| 8 | 1,7 |
| 9 | 0,55 |
| 10 | 5,7 |
| 11 | 5,9 |
| 12 | 1,1 |
| 13 | 8,6 |
| 14 | 2,6 |
| 15 | 0,65 |
| 16 | 13,5 |
| 17 | 23 |
| Théophylline | 30 |

Les composés de l'invention ont en outre été testés sur le modèle de la pleurésie au Zymosan chez le rat (Tarayre et al. Pharmacol. Res., 21, 385, 1989) selon le protocole suivant :
. Technique :
   On injecte dans la cavité pleurale de rats mâles Sprague Dawley légèrement anesthésiés à l'éther, 10 mg de Zymosan sous un volume de 0,15 ml à NaCl 0,9 % stérile. Les animaux sont sacrifiés 1 heure après et l'exsudat pleural est prélevé et mesuré.
. Traitement :
   Les animaux sont à jeun de nourriture depuis la veille. Les produits sont administrés p.o., une heure avant l'injection de l'agent phlogogène, à la dose de 100 mg/kg.

Les résultats obtenus sont reportés dans le tableau IV ci-dessous.

**TABLEAU - IV**

| Composé n° | Pleurésie Zymosan volume exsudat pleural |
|---|---|
| 5 | - 21 % |
| 6 | - 27 % |
| 7 | - 44 % |
| 8 | + 4 % |
| 9 | - 29 % |
| 10 | - 47 % |
| 11 | - 33 % |
| 12 | - 31 % |
| 13 | - 43 % |
| 14 | - 14 % |
| 15 | - 5 % |

### 2 - Applications thérapeutiques

Les composés de l'invention sont des bronchodilatateurs qui peuvent être utilisés pour le traitement des maladies telles que les bronchopneumopathies obstructives chroniques, l'insuffisance respiratoire, l'emphysème.

Les compositions pharmaceutiques peuvent être sous forme appropriée pour l'administration par voie orale, rectale, parentérale, ou locale, par exemple sous la forme de capsules, comprimés, granulés, gélules ou solutés liquides, sirops ou suspensions buvables, aérosols ou solutions pulvérisables, et contenir les excipients appropriés.

La posologie quotidienne peut aller de 50 à 1000 mg.

## Revendications

1. Les dérivés de dihydro-1,2 oxo-2 amino-3 quinoxaline de formule générale I 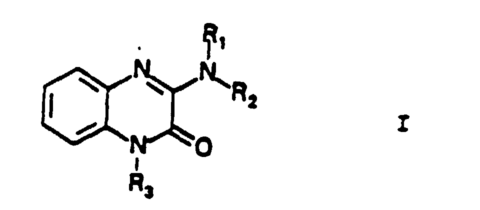 dans laquelle
R₁ et R₂ représentent, indépendamment l'un et l'autre, l'hydrogène ou un radical alcoyle en C₁-C₄ linéaire ou ramifié,
R₃ représente un groupement alcoyle, alcényle ou hydroxyalcoyle, en C₃-C₆ linéaire ou ramifié,
leurs sels avec des acides pharmaceutiquement acceptables.

2. Dérivés de formule générale I selon la revendication 1, caractérisés par le fait qu'ils sont choisis parmi :
- n. propyl-1 dihydro-1,2 oxo-2 amino-3 quinoxaline
- n. propyl-1 dihydro-1,2 oxo-2 méthylamino-3 quinoxaline
- n. propyl-1 dihydro-1,2 oxo-2 diméthylamino-3 quinoxaline
- n. butyl-1 dihydro-1,2 oxo-2 amino-3 quinoxaline
- n. butyl-1 dihydro-1,2 oxo-2 méthylamino-3 quinoxaline
- n. butyl-1 dihydro-1,2 oxo-2 diméthylamino-3 quinoxaline
- allyl-1 dihydro-1,2 oxo-2 amino-3 quinoxaline
- allyl-1 dihydro-1,2 oxo-2 méthylamino-3 quinoxaline
- allyl-1 dihydro-1,2 oxo-2 diméthylamino-3 quinoxaline
- prenyl-1 dihydro-1,2 oxo-2 méthylamino-3 quinoxaline
- prenyl-1 dihydro-1,2 oxo-2 diméthylamino-3 quinoxaline
- (méthyl-3 hydroxy-3) butyl-1 dihydro-1,2 oxo-2 amino-3 quinoxaline
- (méthyl-3 hydroxy-3) butyl-1 dihydro-1,2 oxo-2 méthylamino-3 quinoxaline,
sous la forme de leur base ou de sels thérapeutiquement acceptables.

3. Procédé de préparation d'un composé selon l'une des revendications 1 et 2, caractérisé en ce que l'on traite un dérivé de l'acide dihydro-1,2 oxo-2 quinoxaline carboxylique-3 de formule générale III 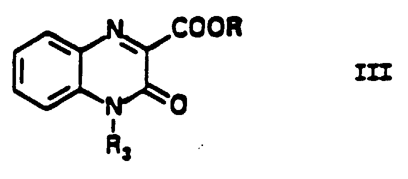 pour laquelle R représente un atome d'hydrogène et R₃ est défini précédemment, par le diphényl phosphoryl azide dans le tertiobutanol, on alcoyle optionnellement le dérivé formé, on l'hydrolyse en milieu acide, puis on alcoyle optionnellement le dérivé obtenu après hydrolyse.

4. Procédé selon la revendication 3, caractérisé en ce que la réaction avec le diphényl phosphoryl azide dans le tertiobutanol est effectuée entre 50°C et la température d'ébullition du tertiobutanol.

5. Procédé selon la revendication 3, caractérisé en ce que la réaction d'alcoylation est effectuée par action d'un réactif R₁Y,
. R₁ représentant un groupe alcoyle en C₁-C₄ linéaire ou ramifié,
. Y représentant un groupement labile tel que Cl, Br, I, mésylate, tosylate, ou d'un dialcoyle C₁-C₂ sulfate.

6. Procédé selon la revendication 5, caractérisé en ce que l'alcoylation est réalisée dans des conditions de transfert de phase en utilisant un milieu biphasique constitué d'un solvant tel que le tétrahydrofurane, une phase aqueuse telle que soude ou potasse et un catalyseur tel que le chlorure de benzyl triéthyl ammonium.

7. Procédé de préparation d'un composé selon la revendication 3, caractérisé en ce que l'étape d'hydrolyse acide est effectuée dans un acide tel que l'acide chlorhydrique alcoolique à une température comprise entre la température ambiante et la température d'ébullition du solvant ou l'acide trifluoroacétique à température ambiante.

8. A titre de médicaments nouveaux, utiles par exemple dans le traitement des maladies telles que les bronchopneumopathies obstructives chroniques, l'insuffisance respiratoire, l'emphysème, les composés définis selon l'une des revendications 1 et 2.

9. Compositions pharmaceutiques caractérisées en ce qu'elles contiennent comme principe actif au moins un composé selon l'une des revendications 1 et 2 associé à un support pharmaceutique inerte.

## Patentansprüche

1. 1,2-Dihydro-2-oxo-3-amino-chinoxalin-Derivate der allgemeinen Formel 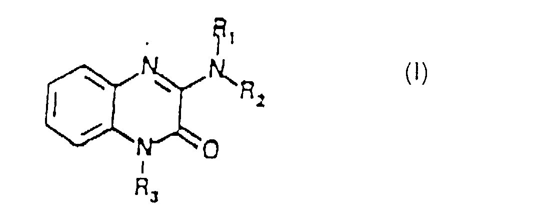 worin bedeuten:
R₁ und R₂ unabhängig voneinander jeweils Wasserstoff oder einen linearen oder verzweigten C₁-C₄-Alkylrest und
R₃ eine lineare oder verzweigte C₃-C₆-Alkyl-, -Alkenyl- oder -Hydroxyalkyl-Gruppe,
und ihre Salze mit pharmazeutisch akzeptablen Säuren.

2. Derivate der allgemeinen Formel (I) nach Anspruch 1, dadurch gekennzeichnet, daß sie ausgewählt werden aus der Gruppe:
- 1-n-Propyl-1,2-dihydro-2-oxo-3-amino-chinoxalin
- 1-n-Propyl-1,2-dihydro-2-oxo-3-methylamino-chinoxalin
- 1-n-Propyl-1,2-dihydro-2-oxo-3-dimethylamino-chinoxalin
- 1-n-Butyl-1,2-dihydro-2-oxo-3-amino-chinoxalin
- 1-n-Butyl-1,2-dihydro-2-oxo-3-methylamino-chinoxalin
- 1-n-Butyl-1, 2-dihydro-2-oxo-3-dimethylamino-chinoxalin
- 1-Allyl-1,2-dihydro-2-oxo-3-amino-chinoxalin
- 1-Allyl-1,2-dihydro-2-oxo-3-methylamino-chinoxalin
- 1-Allyl-1,2-dihydro-2-oxo-3-dimethylamino-chinoxalin
- 1-Prenyl-1,2-dihydro-2-oxo-3-methylamino-chinoxalin
- 1-Prenyl-1,2-dihydro-2-oxo-3-dimethylamino-chinoxalin
- (3-Methyl-3-hydroxy)-1-butyl-1,2-dihydro-2-oxo-3-amino-chinoxalin
- (3-Methyl-3-hydroxy)-1-butyl-1,2-dihydro-2-oxo-3-methylamino-chinoxalin
in Form ihrer Base oder ihrer therapeutisch akzeptablen Salze.

3. Verfahren zur Herstellung einer Verbindung nach einem der Ansprüche 1 und 2, dadurch gekennzeichnet, daß man ein Derivat der 1,2-Dihydro-2-oxo-chinoxalin-3-carbonsäure der allgemeinen Formel 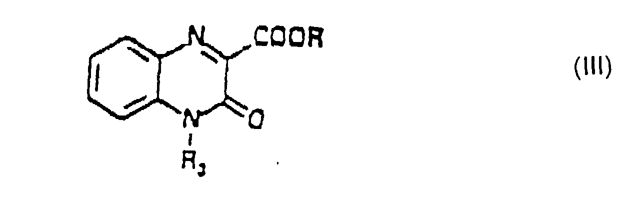 worin R für ein Wasserstoffatom steht und R₃ wie oben definiert ist,
mit Diphenylphosphorylazid in tert-Butanol behandelt, das gebildete Derivat gegebenenfalls alkyliert, es in einem sauren Medium hydrolysiert und dann das nach der Hydrolyse erhaltene Derivat gegebenenfalls alkyliert.

4. Verfahren nach Anspruch 3, dadurch gekennzeichnet, daß man die Umsetzung mit Diphenylphosphorylazid in tert-Butanol zwischen 50°C und der Siedetemperatur des tert-Butanols durchführt.

5. Verfahren nach Anspruch 3, dadurch gekennzeichnet, daß die Alkylierungsreaktion unter Verwendung eines Reagens R₁Y, worin bedeuten:
- R₁ eine lineare oder verzweigte C₁-C₄-Alkylgruppe und
- Y eine labile Gruppe wie Cl, Br, I, Mesylat, Tosylat,
oder eines C₁-C₂-Dialkyl-sulfats durchgeführt wird.

6. Verfahren nach Anspruch 5, dadurch gekennzeichnet, daß die Alkylierung unter Phasenumwandlungsbedingungen durchgeführt wird unter Verwendung eines biphasischen Mediums, das besteht aus einem Lösungsmittel wie Tetrahydrofuran, einer wäßrigen Phase wie Natriumhydroxid oder Kaliumhydroxid, und einem Katalysator wie Benzyltriethylammoniumchlorid.

7. Verfahren zur Herstellung einer Verbindung nach Anspruch 3, dadurch gekennzeichnet, daß die Stufe der sauren Hydrolyse in einer Säure wie alkoholischer Chlorwasserstoffsäure bei einer Temperatur zwischen Umgebungstemperatur und der Siedetemperatur des Lösungsmittels oder in Trifluoressigsäure bei Umgebungstemperatur durchgeführt wird.

8. Die Verbindungen nach einem der Ansprüche 1 und 2 als neue Arzneimittel, die beispielsweise für die Behandlung von Erkrankungen, wie chronischen Obstruktions-Bronchopneumopathien, Atmungsinsuffizienz und Emphysem verwendbar sind.

9. Pharmazeutische Zusammensetzungen, dadurch gekennzeichnet, daß sie als Wirkstoff (aktives Prinzip) mindestens eine Verbindung nach einem der Ansprüche 1 und 2, assoziiert mit einem pharmazeutisch inerten Träger, enthalten.

## Claims

1. 1,2-Dihydro-2-oxo-3-aminoquinoxaline derivatives of general formula I 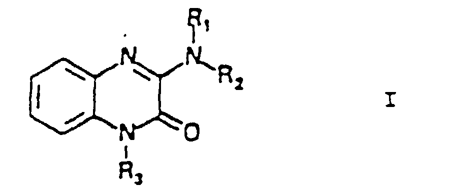 in which
R₁ and R₂ represent, independently of each other, hydrogen or a linear or branched C₁-C₄ alkyl radical,
R₃ represents a linear or branched C₃-C₆ alkyl, alkenyl or hydroxyalkyl group,
and the salts thereof with pharmaceutically acceptable acids.

2. Derivatives of general formula I according to Claim 1, characterized in that they are chosen from:
- 1-n-propyl-1,2-dihydro-2-oxo-3-aminoquinoxaline
- 1-n-propyl-1,2-dihydro-2-oxo-3-methylaminoquinoxaline
- 1-n-propyl-1,2-dihydro-2-oxo-3-dimethylaminoquinoxaline
- 1-n-butyl-1,2-dihydro-2-oxo-3-aminoquinoxaline
- 1-n-butyl-1,2-dihydro-2-oxo-3-methylaninoquinoxaline
- 1-n-butyl-1,2-dihydro-2-oxo-3-dimethylaminoquinoxaline
- 1-allyl-1,2-dihydro-2-oxo-3-aminoquinoxaline
- 1-allyl-1,2-dihydro-2-oxo-3-methylaminoquinoxaline
- 1-allyl-1,2-dihydro-2-oxo-3-dimethylaminoquinoxaline
- 1-prenyl-1,2-dihydro-2-oxo-3-methylaminoquinoxaline
- 1-prenyl-1,2-dihydro-2-oxo-3-dimethylaminoquinoxaline
- 1-(3-methyl-3-hydroxy)butyl-1,2-dihydro-2-oxo-3-aminoquinoxaline
- 1-(3-methyl-3-hydroxy)butyl-1,2-dihydro-2-oxo-3-methylaminoquinoxaline,
in the form of the base thereof or in the form of therapeutically acceptable salts.

3. Process for the preparation of a compound according to either of Claims 1 and 2, characterized in that a 1,2-dihydro-2-oxoquinoxaline-3-carboxylic acid derivative of general formula III 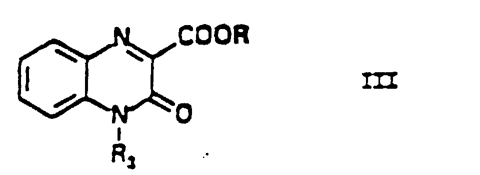 for which R represents a hydrogen atom and R₃ is defined above, is treated with diphenylphosphoryl azide in tert-butanol, the derivative formed is optionally alkylated, it is hydrolysed in an acidic medium and the derivative obtained after hydrolysis is then optionally alkylated.

4. Process according to Claim 3, characterized in that the reaction with diphenylphosphoryl azide in tert-butanol is carried out between 50°C and the boiling point of tert-butanol.

5. Process according to Claim 3, characterized in that the alkylation reaction is carried out by the action of a reactant R₁Y,
· R₁ representing a linear or branched C₁-C₄ alkyl group,
· Y representing a labile group such as Cl, Br, I, mesylate, tosylate or a C₁-C₂ dialkyl sulphate.

6. Process according to Claim 5, characterized in that the alkylation is performed under phase transfer conditions using a two-phase medium consisting of a solvent such as tetrahydrofuran, an aqueous phase such as sodium hydroxide or potassium hydroxide and a catalyst such as benzyltriethylammonium chloride.

7. Process for the preparation of a compound according to Claim 3, characterized in that the acidic hydrolysis step is carried out in an acid such as alcoholic hydrochloric acid, at a temperature between room temperature and the boiling point of the solvent, or trifluoroacetic acid at room temperature.

8. By way of novel medicines which are useful, for example, in the treatment of diseases such as chronic obstructive bronchopneumopathies, respiratory insufficiency and emphysema, the compounds defined according to either of Claims 1 and 2.

9. Pharmaceutical compositions, characterized in that they contain, as active principle, at least one compound according to either of Claims 1 and 2, combined with an inert pharmaceutical vehicle.
